# EUROPEAN PATENT APPLICATION

(11) **EP 1 234 578 A2**
(43) Date of publication of application: **28.08.2002**
(21) Application number: 02251262.8
(22) Date of filing: 25.02.2002
(51) Int. Cl.: A61K 35/78, A61K 35/26, A61P 35/00, A61P 31/12

(54) **Antitumor and antiviral medications and method for producing the same**

(30) Priority: 26.02.2001 JP 2001050775
(71) Applicant: Lymphotec Inc., Tokyo (JP)
(72) Inventor: Tei, Munetetsu, Tokyo (JP)
(74) Representative: Green, Mark Charles

(57) **Abstract**

Antitumor and antiviral medications of curative or preventive efficacy on tumors or cancers can be produced by activating lymphocytes collected or proliferated and inducing heat shock proteins having a prescribed molecular weight in the activated lymphocytes. By heating the activated lymphocytes or adding galenical extract of crude drugs or their compounds to the activated lymphocytes, the heat shock proteins having the desired molecular weight of the order of 70 kDa can be induced in the activated lymphocytes. Thus, the medications remarkably effective in preventing and treating various types of cancers, tumors and viral infections can be obtained.

## Description

This invention relates to antitumor and antiviral medications of curative or preventive efficacy on tumors or cancers, and a method for producing the medications. More particularly, this invention relates to antitumor and antiviral medications produced by inducing heat shock proteins in activated lymphocytes.

Induction of heat shock proteins (hereinafter occasionally abbreviated as "HSP") of various molecular weights by application of heat to cells or chemical treatment has been known. It has also been known that lymphocytes can be proliferated by solid-phase anti-CD3 antigens and interleukin 2, and peripheral-blood derived lymphocytes can be proliferated by anti-CD3 antigens and interleukin 2.

Japanese Patent Publication No. 03-080076 discloses that the lymphocytes thus proliferated have an antitumor effect. It has been further recognized that autologous lymphocytes have a definite antitumor activity, and combination chemotherapy using lymphocytes proliferated by the interleukin 2 is effective against lung cancer to a degree.

However, it has not yet been provided whether or not all cells inducing the aforementioned HSP have antitumor activity. Moreover, any of the lymphocytes mentioned above cannot bring about sufficient anticancerous effects. Thus, there has been a need for antitumor and antiviral medications with a curative effect on tumors or cancers.

The present invention seeks to provide antitumor and antiviral medications of considerably curative efficacy on tumors or cancers.

The present invention also seeks to provide effective antitumor and antiviral medications taking effect on various sorts of tumors with high possibility by the antitumor aptitude of activated lymphocytes.

The present invention also seeks to provide a method for producing antitumor and antiviral medications of considerably curative efficacy on tumors or cancers.

To attain the objects described above according to the present invention, there is provided antitumor and antiviral medications produced by activating lymphocytes and inducing heat shock proteins in the activated lymphocytes. The induction of the heat shock proteins in the activated lymphocytes is achieved by adding galenical extract of crude drugs or their compounds to the activated lymphocytes or heating the activated lymphocytes to increase expression of heat shock proteins of a certain molecular weight, thus bringing about an antitumor and antiviral effect. As one example, the heat shock proteins having a prescribed molecular weight, preferably of the order of 70 kDa, are induced in the activated lymphocytes.

The means for inducing the heat shock proteins having the prescribed molecular weight as mentioned above in the activated lymphocytes comprises a step of heating or warming the activated lymphocytes under a regular condition. The induction of the heat shock proteins for producing the antitumor and antiviral medications according to the invention is accomplished by adding galenical extract of crude drugs such as Rauwolfia serpentina, linderae radix extract, safflower extract and Scutellariae Radix or their compounds to a culture solution of the activated lymphocytes. As the chief ingredient of the antitumor medications of the invention, there may be used reserpine alone or reserpine compound consisting of an extracted galenical compound such as Rauwolfia serpentina extract.

The present invention further provides a method for producing antitumor and antiviral medications, which comprises activating lymphocytes and inducing heat shock proteins in the activated lymphocytes. The method for producing the medications according to the invention is practiced by using means for inducing the heat shock proteins having the prescribed molecular weight as mentioned above in the activated lymphocytes comprises the step of warming the activated lymphocytes under a regular condition. In combination with the means for inducing the heat shock proteins having the prescribed molecular weight, the producing method of the invention may use means for adding the galenical extract of crude drugs such as Rauwolfia serpentina, linderae radix extract, safflower extract and Scutellariae Radix or their compounds to a culture solution of the activated lymphocytes. Having a combination of administering the activated lymphocytes warmed up and directly administering the crude drugs or their compounds to a living organism, the heat shock proteins having the prescribed molecular weight of the order of 70 kDa can be induced in the activated lymphocytes.

Other and further objects of this invention will become obvious upon an understanding of the embodiments described above or will be indicated in the appended claims, and various advantages not referred to herein will occur to one skilled in the art upon employment of the invention in practice.

One embodiment of the present invention will be described hereinafer in detail.

Heat shock protein (HSP) is immune material contained in large quantities in cells of mammals such as a human and an animal. The aforesaid HSP is one kind of proteins, which is easy to react with heat. By heating the cells at a predetermined temperature to impart a stimulus (stress) to the cells, HSP can be induced on the surfaces of the cells. On recent investigation, it has been found that HSP is induced by stimulating the cells with chemical treatment. In any case, the cells inducing HSP has more great immunity function, and are possibly effective particularly against tumors or cancers. According to the invention, the antitumor and antiviral effects can be remarkably increased by activating lymphocytes and heating the lymphocytes thus activated under a prescribed condition. Alternatively, the lymphocytes may be activated by adding the galenical extract of crude drugs or their compounds contained therein thereto. Consequently, the heat shock proteins (HSP) having a prescribed molecular weight, preferably of the order of 70 kDa, can be induced The procedure of producing antitumor and antiviral medications according to the invention will be described hereinafter in more detail.

### [Collecting of lymphocyte cells]

Typically, lymphocytes may be collected from a mouse or human. The lymphocytes derived from the mouse may generally be segregated from the lymph fluid drawn from the spleen or thymus of the mouse. To obtain the lymphocytes derived from the human, it is easy and convenient to segregate the lymphocytes from the lymph fluid in the peripheral blood drawn from the human. It is desirable to draw the peripheral blood from the vein of the human. The amount of the peripheral blood drawn from the human is generally on the order of 0.01 ml to 100 ml, but not specifically limited thereto.

Having regard to a burden imposed on a donor and labor taken in collecting the peripheral blood and a troublesome operation in segregating the lymphocyte cells, it is recommendable to collect the blood of the order of 5 ml to 50 ml in one operation. The drawn blood of the order of 10 ml to 20 ml is more desirable. Further, it is more desirable to add heparin or citric acid to the collected blood in order to prevent the blood from coagulating. Alternatively, the segregation of the lymphocyte cells from the collected blood may be fulfilled by a known lymphocyte cell segregating method such as a discontinuous density gradient centrifugation method using cane sugar or lymph separating medium on the market.

### [Culture and activation of lymphocytes]

If the lymphocytes collected from the aforementioned spleen, thymus or peripheral blood are enough, the lymphocytes may be merely activated, and thus, the lymphocytes do not need proliferation in theory. However, it is practically difficult to collect a sufficient quantity of lymphocytes in the aforesaid manner. For this reason, the lymphocytes may be cultured to proliferate and activated in vitro. The cultivation of the lymphocytes may be performed by a common cell-cultivating method, in which the lymphocyte cells are cultivated in an incubator having a CO₂ concentration of the order of 1 to 10%, preferably 5%, at an atmosphere temperature of approximately 37°C.

More effective cultivation and proliferation of the lymphocytes collected can be achieved in the presence of interleukin 2 and anti-CD3 antibody in combination. In the case of using the interleukin 2 and anti-CD3 antibody, the cultivation of the lymphocytes may be carried out by permitting the lymphocyte cells to float in a culture medium containing the interleukin 2 and placing the culture medium in the incubator containing the anti-CD3 antibody in a solid phase. In this case, as occasion demands, various types of mitogen growth factor or activating factors to cultivate and proliferate the lymphocyte cells.

The anti-CD3 antibody adopted herein may be chosen from antibodies capable of accelerating the cultivation and proliferation of the lymphocytes, and is not specifically limited. The anti-CD3 antibody applied for stimulating the lymphocyte cells may be yielded in an animal or cells by use of refined CD3 molecules, there may be used commercially available OKT-3 antibody (produced by Ortho Pharmaceutical Corp), which excels in stability and cost.

It is desirable to use the anti-CD3 antibody in a solid phase from the point of view of the proliferating efficiency and easiness in handling of the lymphocyte cells. For congealing the anti-CD3 antibody to the solid state, there may be used a culture vessel of glass, polyurethane, polyolefine, polystyrene, or the like. A cell cultivating flask made of plastic, which is available on the market, may be used. The size of the culture vessels or flask is arbitrarily determined.

The solidification of the aforesaid anti-CD3 antibody is performed by adding the diluted solution of anti-CD3 antibody into a solidifying appliance and permitting it to stand for 2 to 24 hours in an atmosphere at a temperature of approximately 4°C to 37°C. It is desirable to dilute the anti-CD3 antibody with a buffer saline solution such as sterilized Dulbecco's phosphate buffered saline solution into concentrations of 1 to 30 µ g/ml. After solidification, the solid-phase anti-CD3 antibody thus diluted is preserved in a cold room or refrigerator (4°C) until use, and separated from a fluid part of the solution or, if necessary, rinsed with a buffer saline solution such as Dulbecco's phosphate buffered saline at room temperature.

In order to increase the proliferating efficiency of the anti-CD3 antibody, it is more desirable to add interleukin 2 to the aforementioned culture medium. In this case, there may be used commercially available interleukin 2 so as to have the culture medium of a concentration of 1 to 2000 U/ml. In use, the interleukin 2 may be dissolved in a culture medium solution in general use such as water, a physiological saline solution, Dulbecco's phosphate buffered saline, RPMI-1640, DMEM, IMDM, AIM-V, or the like, It is desirable to preserve the dissolved interleukin 2 under refrigeration.

In addition, the culture medium used herein is not specifically limited to the above. Any of culture mediums may be used for cultivating the lymphocytes as far as it is congenial to cultivation of lymphocyte cells. For example, as the culture medium used herein, there may be used an organism-derived culture solution such as a blood serum, and a synthetic medium made by adding amino acid, vitamin, nucleic acid base and so forth. Also, there may be enumerated RPMI-1640, DMEM, and IMDM as the culture medium. Of these, RPMI-1640 is most suited in particular for cultivating the lymphocytes used herein. Although a commercially available culture medium may be used, a culture medium with addition of normal human serum excels in a proliferating effect and is far better than the marketed culture medium.

### [Induction of HSP / Heating of lymphocyte cells]

Heating of the lymphocyte cells is effective for inducing heat shock proteins of a specific molecular weight in the activated lymphocytes as noted above. The heat shock proteins induced herein have an excellent antitumor function of treating various cancers, preventing recurrence of the cancers, and improving an antiviral function. For assuring these antitumor and antiviral functions, the heat shock proteins having a molecular weight in the range of 60 to 80 kDa, preferably 70 kDa, is desirably used. To obtain HSP having the aforesaid molecular weight, the heating of the lymphocytes proliferated may be selectively performed under the prescribed conditions of temperature and time determined in accordance with a warming vessel and the condition of the lymphocytes.

However, since a common cultivating temperature for lymphocytes is generally 37°C, the lymphocytes should be heated in the shortest possible time at an atmosphere temperature above the common cultivating temperature so as not to annihilate the lymphocyte tissue. That is, when the heating atmosphere temperature is lower than 38°C, the heat shock proteins become difficult to induce, and contrarily, the heating atmosphere temperature over 50°C possibly attacks the lymphocyte cells. Accordingly, in general, it is recommendable to warm up the activated lymphocyte cells at temperatures of 38°C to 50°C, preferably in the range of 42°C to 45°C, for several seconds to several hours, for instance, 5 seconds to 6 hours, preferably for 10 minutes to 60 minutes.

As the warming vessel for heating the lymphocytes, there may be used a plastic centrifuging tube, culture bag, transfusion bag or any other suitable vessels capable of keeping the lymphocytes in an aseptic condition.

### [Induction of HSP / extraction of crude drugs]

As another measure for inducing the heat shock proteins having a prescribed molecular weight in the activated lymphocytes, galenical extracts of crude drugs or their compounds may be added to the culture solution of the lymphocytes collected or proliferated in the aforementioned manner. The crude drugs applicable to this measure include Rauwolfia serpentina, linderae radix extract, safflower extract and Scutellariae Radix and the compounds contained in these crude drugs.

To be more specific, the galenical extract of crude drugs can be produced by comminuting the crude drug of several grams to several tons, soaking the comminuted crude drug in organic solvent or a combination of water and organic solvent, and being allowed to stand for several hours to several weeks. The galenical extract is solidified or concentrated by a freeze drying method, redistilling method or other adequate methods. There may be adopted a tentative process of producing the galenical extract of crude drugs by cut the crude drug with scissors into a crude drug piece of about 10 grams and soaking the crude drug piece in aqua distillata containing about 50% of ethanol for three days.

As the compound of the crude drug, reserpine may be used by way of example. From the galenical extract thus obtained from the crude drugs, an extract concoction having a concentration of, for example, several nanograms to several hundred milligrams is obtained. The extract concoction thus obtained is added to the collected lymphocytes or proliferated lymphocytes, thus to induce the desired heat shock proteins on the activated lymphocytes.

The synergistic effect brought about by concurrently heating the activated lymphocytes and adding the galenical extract of crude drugs to the activated lymphocytes can accelerate the induction of the heat shock proteins having a molecular weight in the range of 60 to 80 kDa, preferably 70 kDa with high efficiency. Moreover, by combining administaton of the activated lymphocyte heated or warmed up to a living body and subcutaneous administration of the crude drugs or their compounds directly to the living body, the activated lymphocytes administered to the living body more actively accelerate the induction of the heat shock proteins having a molecular weight of about 70 kDa.

### [Embodiment 1]

### [Preparation of lymphocytes heated]

Lymphocytes were collected from the spleen of a C57BL/6 mouse by first aseptically taking out the spleen, and mashing up the spleen with a pair of tweezers in a culture dish having a culture solution containing blood serum from cattle embryo. The solution including the flaked spleen was filtrated through a mesh to obtain lymphocyte suspension. The lymphocyte suspension was transferred by 50ml to a centrifuging tube with a pipette and centrifuged at 1500 rpm for 10 minutes.

After centrifugation, supernatant fluid was removed, and a culture solution was added to obtain 2 × 10⁶/ml of solution. The resultant solution thus obtained was transferred to a culture dish containing solid-phase anti-CD3 antibody from a mouse and further cultivated for 4 days on a continuing basis. The resultant solution was further rinsed with phosphate buffer (occasionally abbreviated as PBS) two times, thus to prepare lymphocytes before heating treatment.

### [Embodiment 2]

### [Measurement of antitumor effect]

After cultivating EL-4 cells (mouse-derived cancer cells) at 37°C in an atmosphere of 5% carbon dioxide gas in a flask for 3 days, 50 ml of culture solution containing El-4 cells were transferred to a centrifuging tube with a pipette and centrifuged at 1500 rpm for 10 minutes. After centrifugation, supernatant fluid was removed, and pH-adjusted RPMI1640 culture medium was added thereto, and brought into suspension. Then, the resultant solution thus obtained was again centrifuged at 1500 rpm for 10 minutes, and thereafter, supernatant fluid was removed. Again, supernatant fluid was removed, and the pH-adjusted RPMI1640 culture medium prepared previously was added thereto, and brought into suspension. Into the abdominal cavity of each C57BL/6 mouse, 5 × 10⁴ of EL-4 cells in the ultimate solution resultantiy obtained was injected to introduce the cancer cells thereinto.

After introducing the EL-4 cells, 3 X 10⁶ of activated lymphocytes treated with heat, which were prepared in Example 1 described above were administered intravenously with administering subcutaneously reseipine thereto. To each mouse of a subject group, reserpine was subcutaneously given by 200 µg/kg every four days together with the untreated lymphocytes, and to another control group composed of twelve mice, only the reserpine was given by 200 µg/kg every four days. The reserpine of Rauwolfia sezpentina extract was used in this embodiment, but it may be made from a compound extracted from Rauwolfia serpentina or other compounds. Although the reserpine solely may be used as mentioned above, but it may be used as a secondary agent with other primary additives.

After that time, the survival rate of mice was observed with time to evaluate the antitumor effects of the activated lymphocytes treated with heat and the reserpine. The results of the evaluation are shown in Table 1 below. As is evident from Table 1, even the administration of reserpine alone brings about an antitumor effect, but the administration of reserpine in combination with the lymphocytes treated with heat can produce more excellent antitumor effect, that is, it can enhance the antitumor ability

**Table 1**

| (Number of surviving mice) | | | | | | | | | | | | | |
|---|---|---|---|---|---|---|---|---|---|---|---|---|---|
| Lapsed days | 23 | 24 | 25 | 26 | 27 | 28 | 29 | 30 | 31 | 32 | 33 | 34 | 35 |
| Control Group | 12 | 11 | 11 | 10 | 9 | 7 | 5 | 2 | 0 | 0 | 0 | 0 | 0 |
| Heated Lymphocytes + Reserpine | 12 | 12 | 12 | 12 | 12 | 12 | 12 | 12 | 11 | 8 | 8 | 7 | 5 |
| Untreated Lymphocytes + Reserpine | 12 | 12 | 12 | 12 | 12 | 10 | 9 | 7 | 6 | 6 | 5 | 3 | 3 |
| Resrpine | 12 | 12 | 12 | 11 | 11 | 11 | 9 | 8 | 8 | 6 | 6 | 4 | 3 |

As is apparent from the foregoing description, according to the invention, the antitumor and antiviral medications having a beneficial effect on human can be produced by activating lymphocytes collected or proliferated and heating the activated or proliferated lymphocytes or adding a galenical extract of crude drugs or their compounds to the activated or proliferated lymphocytes. The antitumor and antiviral medications thus produced have a function of inducing heat shock proteins having a prescribed molecular weight, which achieve beneficial antitumor and antiviral effects on human with high possibility. Even in the lymphocytes collected from the peripheral blood of human or activated lymphocytes actively cultivated, the heat shock proteins having the prescribed molecular weight of the order of 70 kDa can be induced. Specifically, the activated lymphocytes having heat shock proteins induced on heating treatment are remarkably effective in preventing and treating various types of cancers and tumors in human bodies. Namely, the activated lymphocytes according to the invention have a beneficial antitumor and antiviral effect on stomach cancer, liver cancer, pancreatic cancer, kidney cancer, carcinoma of the colon and rectum, lung cancer, brain tumor; sarcoma and so on.

Furthermore, the activated lymphocytes according to the invention have an effect of preventing recurrence of cancers and tumors, and preventing and treating various types of infection diseases and autoimmune diseases. The activated lymphocytes according to the invention have a beneficial effect specifically on microbism, viral infections, and so forth. The causative viruses of viral infections are human immunodeficiency virus, EB virus, cytomegalovirus, herpes virus, influenza virus, and so on That is, the medications according to the invention are effective against almost all types of viral infectious diseases caused by known or unforehnown viruses.

According to the invention, the heat shock proteins having a molecular weight in the range of 60 to 80, which have significant antitumor and antiviral functions capable of being performed with high possibility, can be induced by adding the extract from crude drugs such as Rauwolfia serpentina, linderae radix extract, safflower extract and Scutellariae Radix and the compounds contained in these crude drugs to the culture solution of the activated lymphocytes. According to the method of the present invention, the synergistic effect can be brought about by concurrently heating the activated lymphocytes and adding the galenical extract of crude drugs to the activated lymphocytes to accelerate the induction of the heat shock proteins having a molecular weight of 70 kDa. Moreover, by combining administration of the activated lymphocyte subjected heated up to a living body and subcutaneous administration of the crude drugs or their compounds directly to the living body, the activated lymphocytes administered to the living body can more actively accelerate the induction of the heat shock proteins having a molecular weight of about 70 kDa.

## Claims

1. Antitumor and antiviral medications produced by activating lymphocytes and inducing heat shock proteins in said activated lymphocytes.

2. The antitumor and antiviral medications set forth in claim 1, wherein said induced heat shock proteins has a molecular weight of 60 kDa to 80 kDa.

3. The antitumor and antiviral medications set forth in claim 1, wherein said induced heat shock proteins has a molecular weight of 70 kDa.

4. The antitumor and antiviral medications set forth in any of claims 1 to 3, wherein said heat shock proteins are induced by heating said activated lymphocytes.

5. The antitumor and antiviral medications set forth in claim 4, wherein said lymphocytes are heated at temperatures of 38°C to 50°C, thereby to induce said heat shock proteins.

6. The antitumor and antiviral medications set forth in claim 4, wherein said lymphocytes are heated at temperatures of 38°C to 50°C for 5 seconds to 6 hours, thereby to induce said heat shock proteins.

7. The antitumor and antiviral medications set forth in claxm 4, wherein said lymphocytes are heated at temperatures of 42°C to 45°C, thereby to induce said heat shock proteins.

8. The antitumor and antiviral medications set forth in claim 4, wherein said lymphocytes are heated at temperatures of 42°C to 45°C for 10 minutes to 60 minutes, thereby to induce said heat shock proteins.

9. The antitumor and antiviral medications set forth in any of claims 1 to 3, wherein said heat shock proteins are induced by adding galenical extract of crude drug or its compounds to a culture solution of said activated lymphocytes.

10. The antitumor and antiviral medications set forth in claim 9, wherein said crude drug is Rauwolfia serpentina.

11. The antitumor and antiviral medications set forth in claim 10, wherein the compound of said Rauwolfia serpentina is reserpine.

12. The antitumor and antiviral medications set forth in claim 9, wherein said crude drug is linderae radix.

13. The antitumor and antiviral medications set forth in claim 9, wherein said crude drug is safflower extract.

14. The antitumor and antiviral medications set forth in claim 9, wherein said crude drug is Scutellariae Radix.

15. Antitumor medications consisting of reserpine alone or containing reserpine as a chief ingredient.

16. The antitumor and antiviral medications set forth in claim 15, wherein said reserpine is derived from galenical extract from Rauwolfia serpentina.

17. A method for producing antitumor and antiviral medications comprising the steps of activating lymphocytes, and heating said lymphocytes activated, thereby to induce heat shock proteins in said lymphocytes.

18. The method for producing antitumor and antiviral medications set forth in claim 17, wherein said induced heat shock proteins has a molecular weight of 60 kDa to 80 kDa.

19. The method for producing antitumor and antiviral medications set forth in claim 17. wherein said induced heat shock proteins has a molecular weight of 70 kDa.

20. The method for producing antitumor and antiviral medications set forth in any of claims 17 to 19, wherein said lymphocytes are heated at temperatures of 38°C to 50°C, thereby to induce said heat shock proteins.

21. The method for producing antitumor and antiviral medications set forth in any of claims 17 to 19, wherein said lymphocytes are heated at temperatures of 38°C to 50°C for 5 seconds to 6 hours, thereby to induce said heat shock proteins.

22. The method for producing antitumor and antiviral medications set forth in any of claims 17 to 19, wherein said lymphocytes are heated at temperatures of 42°C to 45°C for 10 minutes to 60 minutes, thereby to induce said heat shock proteins.

23. A method for producing antitumor and antiviral medications comprising the steps of activating lymphocytes, and adding galenical extract of crude drug or its compounds to a culture solution of said activated lymphocytes, thereby to induce heat shock proteins in said lymphocytes.

24. The method for producing antitumor and antiviral medications set forth in claim 23, wherein said crude drug is Rauwolfia serpentina, linderae radix extract, safflower extract or Scutellariae Radix.

25. A method for producing antitumor and antiviral medications comprising the steps of activating lymphocytes, and concurrently heating said activated lymphocytes and adding galenical extract of crude drug or its compounds to a culture solution of said activated lymphocytes, thereby to induce heat shock proteins having a molecular weight of 60 kDa to 80 kDa in said lymphocytes.

26. A method for producing antitumor and antiviral medications comprising the steps of activating lymphocytes, heating said activated lymphocytes, and concurrently administering, to a living body, said activated lymphocytes thus heated and galenical extract of crude drug or its compounds, thereby to induce heat shock proteins having a molecular weight of 60 kDa to 80 kDa in said
